# EUROPÄISCHE PATENTSCHRIFT

(11) **EP 0 589 908 B1**
(45) Veröffentlichungstag und Bekanntmachung des Hinweises auf die Patenterteilung: **29.07.1998**
(21) Anmeldenummer: 92910172.3
(22) Anmeldetag: 12.05.1992
(51) Int. Cl.: C07D 277/34, A61K 31/425, A61K 31/435, C07D 417/06

(54) **AMINOALKYLSUBSTITUIERTE THIAZOLIN-2-ONE, IHRE HERSTELLUNG UND VERWENDUNG**
AMINOALKYL-SUBSTITUTED THIAZOLIN-2-ONES, THEIR PREPARATION AND THEIR USE
THIAZOLIN-2-ONES A SUBSTITUTION AMINOALKYLE, LEUR PREPARATION ET LEUR UTILISATION

(30) Priorität: 15.06.1991 DE 4119757
(43) Veröffentlichungstag der Anmeldung: 06.04.1994
(73) Patentinhaber: BASF Aktiengesellschaft, 67063 Ludwigshafen (DE)
(72) Erfinder: RENDENBACH-MUELLER, Beatrice, D-6701 Waldsee (DE); TESCHENDORF, Hans-Juergen, D-6724 Dudenhofen (DE); UNGER, Liliane, D-6700 Ludwigshafen (DE)
(86) Internationale Anmeldenummer: EP9201039
(87) Internationale Veröffentlichungsnummer: WO9222539

(56) Entgegenhaltungen:
- DE-A- 2 521 104

## Beschreibung

Die Erfindung betrifft aminoalkylsubstituierte Thiazolin-2-one, deren Herstellung sowie deren Verwendung bei der Bekämpfung von Krankheiten.

In BE 829 071 werden 4-Aryl-5-aminoalkylthiazolin-2-one mit pharmakologischen Aktivitäten beschrieben.

Es wurde nun gefunden, daß aminoalkylsubstituierte Thiazolin-2-one der Formel I 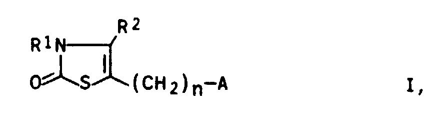 in der
R¹ H oder C₁-C₅-Alkyl bedeutet,
R² H oder C₁-C₅-Alkyl bedeutet,
n eine ganze Zahl von 1 bis 6 ist,
A eine der Gruppen 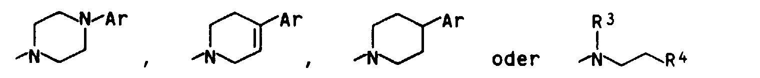 ist, wobei Ar für einen gegebenenfalls einfach durch C₁-C₅-Alkyl, C₁-C₅-Alkoxy, Halogen, Nitro, Hydroxy, C₁-C₅-Alkylthio, Trifluormethyl oder Cyano substituierten Phenylring, einen Pyridyl-, Pyrimidinyl- oder Thienylrest steht, R³ H oder einen C₁-C₅-Alkyl-rest darstellt und R⁴ Phenyl, das gegebenenfalls einfach durch C₁-C₅-Alkyl, C₁-C₅-Alkoxy, Halogen, Hydroxy oder Trifluormethyl substituiert ist, oder einen Thienylrest bedeutet
sowie ihre Tautomere und Salze dieser Verbindungen mit physiologisch verträglichen Säuren interessante pharmakologische Eigenschaften besitzen.

In der Formel I bedeutet R¹ vorzugsweise H, R² vorzugsweise CH₃, n vorzugsweise 2, 3 oder 4 und A vorzugsweise 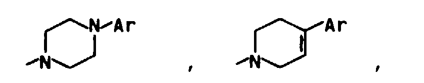 worin Ar einen Phenyl-, Pyridyl- oder Pyrimidinylrest bedeutet, oder 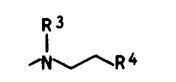 worin R³ C₁-C₃-Alkyl und R⁴ Phenyl oder Thienyl bedeuten.

Die Tautomeren, die sich von den Verbindungen der Formel I ableiten, besitzen die Formel Ia

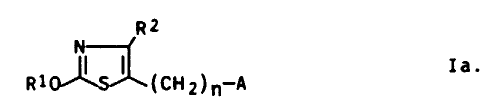

Die Verbindungen der Formel I lassen sich herstellen, indem man
a) ein ω-X-alkylsubstituiertes Thiazolin-2-on der Formel II 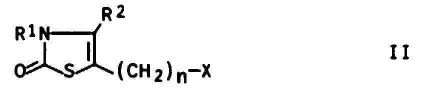 in der R¹, R² und n wie eingangs definiert sind und X für eine Abgangsgruppe wie Chlor, Brom oder R⁵SO₂O- steht [R⁵ = C₁-C₄-Alkyl oder gegebenenfalls durch C₁-C₃-Alkyl oder Halogen substituiertes Phenyl], oder, wenn R¹=H ist, ein Tautomeres dieser Verbindung mit einem Amin der Formel III

   HA III,

   in der A die angegebenen Bedeutungen hat, umsetzt oder
b) ein α-Halogenketon der Formel IV 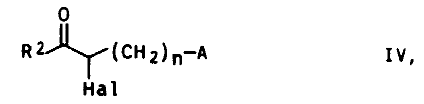 in der R², R³, A und n wie eingangs definiert sind und Hal ein Chlor-, Brom- oder Iodatom darstellt, oder ein halogenwasserstoffsaures Salz dieser Verbindung mit einem Thiocarbamat der Formel V 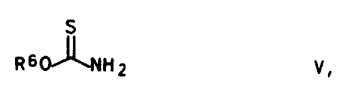 in der R⁶ C1-C5-Alkyl bedeutet oder mit einem Tautomeren dieser Verbindung umsetzt, oder
c) eine Verbindung der Formel VI 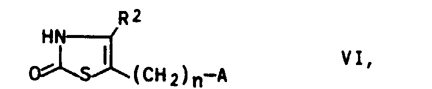 in der R2, A und n wie eingangs definiert sind, mit einem Alkylierungsreagenz R1-X (X = Cl, Be, R⁵SO₂O-) oder R¹₂SO₄ alkyliert und die so erhaltenen Verbindungen gegebenenfalls in ihre Salze mit physiologisch verträglichen Säuren überführt.

Im Falle des Verfahrens a) erfolgt die Umsetzung in der Schmelze, gewünschtenfalls auch in Gegenwart eines Lösungsmittels, z.B. Ethanol, Butanol, Ethylacetat, Tetrahydrofuran, Dimethylformamid, Dimethoxyethan, Toluol oder Xylol bei Temperaturen zwischen Raumtemperatur und dem Siedepunkt des verwendeten Lösungsmittels, vorzugsweise in Gegenwart einer Base wie Natriummethylat, Natriumethylat, Natriumhydrid, Natriumcarbonat, Kaliumcarbonat oder eines Amins, z.B. Pyridin. Gegebenenfalls kann auch die Aminkomponente IV im Überschuß als Reagenz, Base und Lösungsmittel fungieren.

Die Isolierung des Rohprodukts erfolgt in an sich üblicher Weise, z.B. durch Filtration, Abdestillieren des Lösungsmittels oder Extraktion aus dem Reaktionsgemisch. Eine Reinigung der erhaltenen Verbindung erfolgt in üblicher Weise, beispielsweise durch Umkristallisieren aus einem Lösungsmittel, Chromatographie oder Überführen in eine Säureadditionsverbindung.

Die als Ausgangsstoffe verwendeten Thiazolin-2-one der Formel II können beispielsweise durch Halogenierung eines ω-X-substituierten Ketons der Formel VII 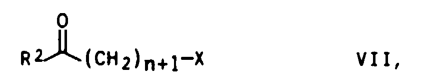 in der R², n und X die angegebenen Bedeutungen haben, und anschließende Umsetzung des halogenierten Ketons mit einem Thiocarbamat der Formel V hergestellt werden.

Die Umsetzungen nach Verfahren b) erfolgen vorzugsweise in einem Lösungsmittel bei Temperaturen zwischen Raumtemperatur und der Siedetemperatur des verwendeten Lösungsmittels, gegebenenfalls in Gegenwart eines säurebindenden Mittels. Als Lösungsmittel können beispielsweise aliphatische Alkohole, Dimethylformamid, Eisessig, Wasser oder ein Lösungsmittelgemisch verwendet werden, als säurebindende Mittel anorganische Basen wie Natrium- oder Kaliumcarbonat oder tertiäre organische Basen wie Triethylamin oder Pyridin. Letztere können, im Überschuß verwendet, gleichzeitig als Lösungsmittel dienen.

Die als Ausgangstoffe verwendeten a-Halogenketone der Formel IV erhält man durch Halogenierung der entsprechenden Ketone der Formel VIII 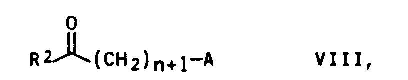 in der R², A und n die angegebenen Bedeutungen haben. Die so erhaltenen Verbindungen der Formel IV brauchen nicht weiter gereinigt zu werden.

Die Umsetzung nach Verfahren c) verläuft nach Methoden, wie sie für die Alkylierung von sekundären Amiden aus der Literatur bekannt sind. Man kann ohne Gegenwart eines Lösungsmittels die Komponenten miteinander verschmelzen. Es ist aber auch möglich, in Gegenwart einen indifferenten Lösungsmittels, z.B. eines aromatischen Kohlenwasserstoffs wie Toluol oder Xylol, eines Ethers wie Tetrahydrofuran oder Dioxan, eines Ketons wie Aceton oder Butanon, eines Amids wie Dimethylformamid oder N-Methylpyrrolidon, eines Nitrils wie Acetonitril, eines halogenierten Kohlenwasserstoffs oder von Dimethylsulfoxid, zu arbeiten. Günstig ist der Zusatz einer Base, wie z.B. Natriummethylat, Natriumethylat, Natriumhydrid, eines Carbonats, eines Hydroxids oder eines tertären Amins wie Triethylamin oder Pyridin.

Gegebenenfalls werden die erhaltenen erfindungsgemäßen verbindungen in ihre Säureadditionsalze mit physiologisch verträglichen Säuren überführt. Als übliche physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere können aus Fortschritte der Arzneimittelforschung Bd. 10, S. 224 f., Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder Propanol, einem halogenierten Kohlenwasserstoff wie Methylenchlorid, einem Ether wie Methyl-t-butylether oder Diisopropylether, einem Keton wie Aceton oder Butanon oder einem Ester wie Essigsäureethylester erhalten. Zur besseren Kristallabscheidung können auch Mischungen der genannten Lösungsmittel verwendet werden. Darüber hinaus können pharmazeutisch vertretbare wäßrige Lösungen von Säureadditonsverbindungen der erfindungsgemäßen Verbindungen I durch Auflösen der freien Basen in einer wäßrigen Säurelösung hergestellt werden.

Die erfindungsgemäßen Verbindungen eignen sich zur Bekämpfung von Krankheiten, insbesondere zur Behandlung von Erkrankungen des zentralen Nervensystems (z.B. Parkinsonismus, Schizophrenie) sowie von erhöhtem Blutdruck. Sie weisen insbesondere wertvolle Eigenschaften als Dopaminagonisten, teilweise mit Selektivität für präsynaptische Dopaminrezeptoren, oder als Dopaminantagonisten auf. Die Verbindungen der Formel I zeigen Affinität zum Dopamin-Rezeptor in Rezeptorbindungstests; sie inhibieren die Motilität an Mäusen (gemessen in Lichtschrankenkäfigen) und beeinflussen das Rotationsverhalten an Ratten mit einseitigen 6-Hydroxydopamin-Läsionen der Substantia nigra (Brain Research 24, 485-493, 1970).

Die Wirkungen der neuen Verbindungen lassen sich im Rezeptorbindungstest zeigen:

Striata von Ratten (Sprague Dawley, Charles River) wurden sofort nach Entnahme in 0,32 M Saccharoselösung (0°C) homogenisiert. Das Homogenat wurde filtriert über Gaze, das Filtrat bei 1000 g zentrifugiert (5 min bei 4°C) und der resultierende Überstand bei 40000 g zentrifugiert (10 min, 4°C). Der Rückstand (Membranen) wurde in Inkubationspuffer (50 mM Tris-HCl, 1 mM MgCl₂ und 0,1 % Ascorbinsäure, pH 7,4) aufgenommen und 20 min bei 37°C inkubiert. Anschließend wurde durch Resuspension und Rezentrifugation 2x mit Inkubationspuffer gewaschen. Die Membranen wurden portionsweise in flüssigem Stickstoff eingefroren.

Die Testansätze (1 ml) setzten sich zusammen aus Membranen (380 µg Protein), 1 nM ³H-ADTN (NEN, Dreieich Germany, spez. Radioaktivität 1,4 TBq/mmol) und 0,1 µM SCH 23390 (totale Bindung) oder a) mit zusätzlich 50 nM Spiperon (unspezifische Bindung) oder b) mit Prüfsubstanz. Die Ansätze wurden als Triplikate hergestellt.

Nach beendeter Inkubation (40 min bei 25°C) wurden die Ansätze über Glasfaserfilter (Whatman GF/B) filtriert und kurz mit eiskaltem Waschpuffer (Tris-HCl, pH 7,4) gewaschen. Die auf den Filtern zurückgehaltene Radioaktivität wurde mittels Flüssigkeitszintillationsmessung bestimmt. Die unspezifische Bindung betrug ca. 40 - 50 % der totalen Bindung.

Die Auswertung der Kompetitionskurven und die Bestimmung der Dissoziationskonstante erfolgte über iterative nichtlineare Regressionsanalyse in Anlehnung an das Programm "Ligand" (Muson und Rodbard: Anal. Biochem. 107, 220, 1980).

Affinität der Prüfsubstanzen zum Dopamin-D₂-Rezeptor

| Beispiel | Ki (nM) |
|---|---|
| 1 | 8 |
| 2 | 15 |
| 7 | 12 |
| 10 | 25 |
| 12 | 6 |
| 13 | 45 |
| 14 | 6 |
| 17 | 40 |

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 500 mg pro Patient und Tag bei oraler Gabe und etwa 1 bis 100 mg pro Patient und Tag bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidatien und/oder Treibgasen verarbeitet werden (vgl. H. Sucker et al: Pharmazeutische Technologie, Thieme-Verlag, Stuttgart, 1978). Die so erhaltenen Applikationen enthalten den Wirkstoff normalerweise in einer Menge von 1 bis 99 Gew.-%.

Die folgenden Beispiele sollen die Erfindung näher erläutern:

### Beispiel 1

### 4-Methyl-5-[3-(1,2,3,6-tetrahydro-4-phenylpyridyl)-propyl]-thiazolin-2-on-hydrochlorid

4,5 g 5-(3-Chlorpropyl)-4-methyl-thiazolin-2-on, 4,6 g 1,2,3,6-Tetrahydro-4-phenylpyridin-hydrochlorid, 2,5 g Natriumcarbonat und eine Spatelspitze Natriumiodid wurden in 10 ml Butanol 10 h unter Rückfluß gekocht. Nach dem Abkühlen wurde das Lösungsmittel entfernt, der Rückstand in Methyl-t-butylether/Wasser verteilt, die organische Phase abgetrennt, nochmals mit Wasser gewaschen und eingeengt. Der Rückstand wurde in 2 N HCI aufgenommen und die Lösung mit Methylenchlorid extrahiert.

Die wäßrige Phase wurde mit 2N NaOH auf pH 8 gestellt und mit Methyl-t-butylether extrahiert. Die vereinigten organischen Phasen wurden getrocknet und eingeengt, der Rückstand in wenig Ethanol gelöst und mit etherischer HCl versetzt. Der nach Zugabe von Methyl-t-butylether ausgefallene Feststoff wurde abgesaugt und im Vakuum getrocknet.
Ausbeute: 2,96 g (36 %) Fp.: 203 - 205°C

Analog Beispiel 1 wurden hergestellt:
2. 4-Methyl-5-[3-(4-pyrid-2-ylpiperazinyl)-propyl]-thiazolin-2-on-dihydrochlorid
   Ausbeute: 23 % Fp.: 244°C (Zers.)
3. 4-Methyl-5-[3-(N-phenethyl-N-n-propylamino)-propyl]-thiazolin-2-on-furmarat
   Ausbeute: 26 % Fp.: 110°C
4. 4-Methyl-5-[3-(4-phenylpiperazinyl)-propyl]-thiazolin-2-on-hydrochlorid
   Ausbeute: 23 % Fp.: 233°C
5. 4-Methyl-5-[3-(N-thien-2-ylethyl-N-n-propylamino)-propyl]-thiazolin-2-on-tartrat
   Ausbeute: 52 % Fp.: 80°C
6. 4-Methyl-5-[3-(4-phenylpiperidinyl)-propyl]-thiazolin-2-on-hydrochlorid
   Ausbeute: 38 % Fp.: 203°C
7. 4-Methyl-5-[3-(4-pyrimidin-2-ylpiperazinyl)-propyl]-thiazolin-2-on-dihydrochlorid
   Ausbeute: 41 % Fp.: 245°C
8. 4-Methyl-5-[3-(4-(3-methoxyphenyl)-piperazinyl)-propyl]-thiazolin-2-on-hydrochlorid
   Ausbeute: 40 % Fp.: 195°C
9. 4-Methyl-5-[3-(4-(4-chlorphenyl)-1,2,3,6-tetrahydropyridyl)-propyl]-thiazolin-2-on-hydrochlorid
   Ausbeute: 37 % Fp.: 250°C
10. 4-Methyl-5-[3-(4-(2-ethylphenyl)-piperazinyl)-propyl]-thiazolin-2-on-dihydrochlorid
   Ausbeute: 25 % Fp.: 230°C
11. 4-Methyl-5-[3-(4-(4-fluorphenyl)-1,2,3,6-tetrahydropyridyl)]-thiazolin-2-on-hydrochlorid
   Ausbeute: 30 % Fp.: 239 - 240°C
12. 4-Methyl-5-[3-(4-(2-methylthiophenyl)-piperazinyl)-propyl]-thiazolin-2-on-hydrochlorid
   Ausbeute: 46 % Fp.: 248 -249°C
13. 4-Methyl-5-[3-(4-(3-trifluormethylphenyl)-piperazinyl)-propyl]-thiazolin-2-on-hydrochlorid
   Ausbeute: 19 % Fp.: 217 - 218°C
14. 4-Methyl-5-[3-(4-(2-ethoxyphenyl)-piperazinyl)-propyl]-thiazolin-2-on-dihydrochlorid
   Ausbeute: 31 % Fp.: 219°C
15. 4-Methyl-5-[3-(4-(3-chlorphenyl)-piperazinyl)-propyl]-thiazolin-2-onhydrochlorid
   Ausbeute: 17 % Fp.: 212°C (Zers.)
16. 4-Methyl-5-[3-(4-(2-cyanophenyl)-piperazinyl)-propyl]-thiazolin-2-on-hydrochlorid
   Ausbeute: 25 % Fp.: 257°C (Zers.)

### Beispiel 17

### 3,4-Dimethyl-5-[3-(4-phenylpiperazinyl)-propyl]-thiazolin-2-on-dihydrochlorid

5 g 4-Methyl-5-[3-(4-phenylpiperazinyl)-propyl]thiazolin-2-on, 3,3 g Kaliumcarbonat, 0,1 g 18-Krone-6 wurden in Dimethylformamid 30 min auf 60°C erwärmt. Anschließend wurden 2,7 g Methyliodid zugetropft und die Reaktionsmischung 3 h zum Rückfluß erhitzt. Nach dem Abkühlen wurde Wasser zugegeben, dreimal mit Methyl-t-butyl-ether extrahiert, die organischen Phasen mit Wasser gewaschen, getrocknet und eingeengt. Der Rückstand wurde in Ethanol gelöst und mit etherischer HCl versetzt. Der nach Abkühlen auskristallisierende Feststoff wurde abgesaugt und getrocknet.
Ausbeute: 3,3 g (51 %) Fp.: 222°C

Beispiele für galenische Applikationsformen:
A) Auf einer Tablettenpresse werden in üblicher Weise Tabletten folgender Zusammensetzung gepreßt.

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil+ (chemisch reine Kieselsäure in |
| | submikroskopisch feiner Verteilung) |
| 6,75 mg | Kartoffelstärke (als 6 %iger Kleister) |

B)

| | |
|---|---|
| 20 mg | Substanz des Beispiels 4 |
| 60 mg | Kernmasse |
| 60 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Kalk. Die so hergestellten Dragees werden anschließend mit einem magensaftresistenten überzug versehen.
C) 10 g Substanz des Beispiels 2 werden in 5000 ml Wasser unter Zusatz von NaCl gelöst und mit 0,1 N NaOH auf pH 6,0 eingestellt, so daß eine blutisotonische Lösung entsteht. Jeweils 1 ml dieser Lösung wird in Ampullen gefüllt und sterilisiert.

## Patentansprüche

1. Aminoalkylsubstituierte Thiazolin-2-one der Formel I 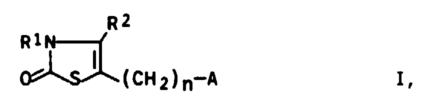 in der
R¹ H oder C₁-C₅-Alkyl bedeutet,
R² H oder C₁-C₅-Alkyl bedeutet,
n eine ganze Zahl von 1 bis 6 ist,
A eine der Gruppen 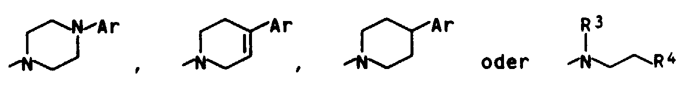 ist, wobei Ar für einen gegebenenfalls einfach durch C₁-C₅-Alkyl, C₁-C₅-Alkoxy, Halogen, Nitro, Hydroxy, C₁-C₅-Alkylthio, Trifluormethyl oder Cyano substituierten Phenylring, einen Pyridyl-, Pyrimidinyl- oder Thienylrest steht, R³ H oder einen C₁-C₅-Alkylrest darstellt und R⁴ Phenyl, das gegebenenfalls einfach durch C₁-C₅-Alkyl, C₁-C₅-Alkoxy, Halogen, Hydroxy oder Trifluormethyl substituiert ist, oder einen Thienylrest bedeutet
sowie ihre Tautomere und Salze dieser Verbindungen mit physiologisch verträglichen Säuren.

2. Verfahren zur Herstellung aminoalkylsubstituierter Thiazolin-2-on-Derivate gemäß Anspruch 1, dadurch gekennzeichnet, daß man
a) ein ω-X-alkylsubstituiertes Thiazolin-2-on der Formel I 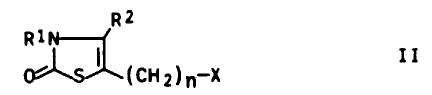 in der R¹, R² und n wie eingangs definiert sind und X für eine Abgangsgruppe wie Chlor, Brom oder R⁵SO₂O-steht [R⁵ = C₁-C₄-Alkyl oder gegebenenfalls durch C₁-C₃-Alkyl oder Halogen substituiertes Phenyl], oder wenn R¹=H ist, ein Tautomeres dieser Verbindung mit einem Amin der Formel III
HA III
in der A die angegebenen Bedeutungen hat, umsetzt oder
b) ein α-Halogenketon der Formel IV 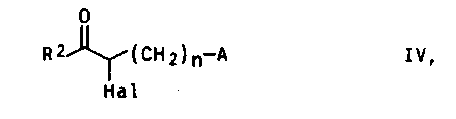 in der R², R³, A und n wie eingangs definiert sind und Hal ein Chlor-, Brom- oder Iodatom darstellt, oder ein halogenwasserstoffsaures Salz dieser Verbindung mit einem Thiocarbamat der Formel V 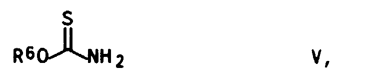 in der R⁶ C₁-C₅-Alkyl bedeutet oder mit einem Tautomeren dieser Verbindung umsetzt, oder
c) eine Verbindung der Formel VI 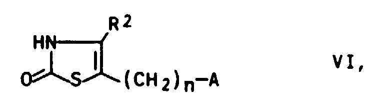 in der R2, A und n wie eingangs definiert sind mit einem Alkylierungsreagenz R1-X (X = Cl, Br, R⁵SO₂O-) ) oder R¹₂SO₄ alkyliert und die so erhaltenen Verbindungen gegebenenfalls in ihre Salze mit physiologisch verträglichen Säuren überführt.

3. Aminoalkylsubstituierte Thiazolin-2-one zur Verwendung bei der Bekämpfung von Krankheiten.

4. Verwendung eines aminoalkylsubstituierten Thiazolin-2-ons der Formel I nach Anspruch 1 zur Herstellung eines Arzneimittels gegen Erkrankungen des zentralen Nervensystems oder Erkrankungen, die mit erhöhtem Blutdruck einhergehen.

## Claims

1. An aminoalkyl-substituted thiazolin-2-one of the formula I 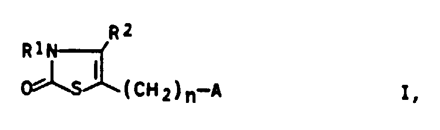 where
R¹ is H or C₁-C₅-alkyl,
R² is H or C₁-C₅-alkyl,
n is an integer from 1 to 6.
A is 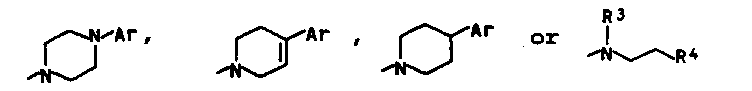 where Ar is phenyl which is unsubstituted or monosubstituted by C₁-C₅-alkyl, C₁-C₅-alkoxy, halogen, nitro, hydroxyl, C₁-C₅-alkylthio, trifluoromethyl or cyano, or is pyridyl, pyrimidinyl or thienyl, R³ is H or C₁-C₅-alkyl, and R⁴ is phenyl which is unsubstituted or monosubstituted by C₁-C₅-alkyl, C₁-C₅-alkoxy, halogen, hydroxyl or trifluoromethyl, or is thienyl,
and its tautomers and salts of these compounds with physiologically tolerated acids.

2. A process for preparing aminoalkyl-substituted thiazolin-2-one derivatives as claimed in claim 1, which comprises
a) reacting an ω-X-alkyl-substituted thiazolin-2-one of the formula II 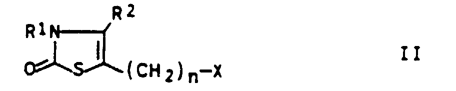 where R¹, R² and n are as defined above, and X is a leaving group such as chlorine, bromine or R⁵SO₂O- [R⁵ = C₁-C₄-alkyl or phenyl which is unsubstituted or substituted by C₁-C₃-alkyl or halogen], or, when R¹ is H, a tautomer of this compound, with an amine of the formula III
HA III,
where A has the stated meanings, or
b) reacting an α-halo ketone of the formula IV 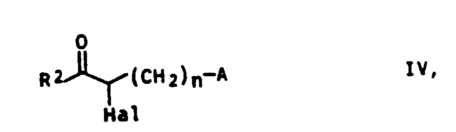 where R², R³, A and n are as defined above, and Hal is chlorine, bromine or iodine, or a hydrohalide of this compound, with a thiocarbamate of the formula V 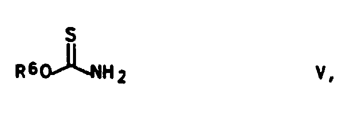 where R⁶ is C₁-C₅-alkyl, or with a tautomer of this compound, or
c) alkylating a compound of the formula VI 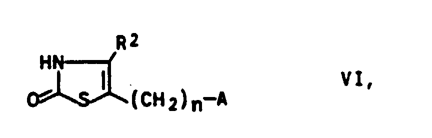 where R², A and n are as defined above, with an alkylating reagent R¹-X (X = Cl, Br, R⁵SO₂O-) or R¹₂SO₄, where appropriate converting the resulting compounds into their salts with physiologically tolerated acids.

3. An aminoalkyl-substituted thiazolin-2-one for use for controlling diseases.

4. The use of an aminoalkyl-substituted thiazolin-2-one of the formula I as claimed in claim 1 for producing a drug for disorders of the central nervous system or disorders associated with elevated blood pressure.

## Revendications

1. Thiazoline-2-ones aminoalkylsubstituées de la formule I 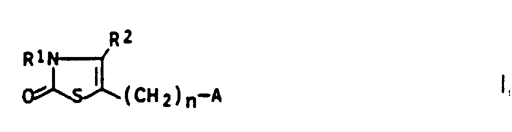 dans laquelle
R¹ représente un atome d'hydrogène ou un radical alkyle en C₁ à C₅,
R² représente un atome d'hydrogène ou un radical alkyle en C₁ à C₅,
n représente un nombre entier dont la valeur varie de 1 à 6,
A représente l'un des radicaux qui suivent: 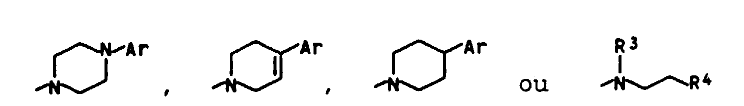 où Ar représente un noyau phényle éventuellement monosubstitué par un radical alkyle en C₁ à C₅, alcoxy en C₁ à C₅, un atome d'halogène, un radical nitro, hydroxyle, alkylthio en C₁ à C₅, trifluorométhyle ou cyano, un radical pyridyle, pyrimidinyle, ou thiényle, R³ représente un atome d'hydrogène ou un radical alkyle en C₁ à C₅, et R⁴ représente un radical phényle qui est éventuellement monosubstitué par un radical alkyle en C₁ à C₅, alcoxy en C₁ à C₅, un atome d'halogène, un radical hydroxyle ou trifluorométhyle, ou bien il représente un radical thiényle,
ainsi que leurs tautomères et les sels de ces composés avec des acides physiologiquement compatibles.

2. Procédé de préparation de dérivés de la thiazoline-2-one aminoalkylsubstitués suivant la revendication 1, caractérisé en ce que
a) on fait réagir une thiazoline-2-one ω-X-alkylsubstituée de la formule II 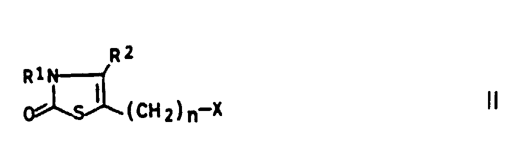 dans laquelle R¹, R² et n possèdent les significations qui leur ont été attribuées dans l'introduction et X représente un groupe sortant, comme un atome de chlore, de brome, ou R⁵SO₂O- [R⁵ = alkyle en C₁ à C₄ ou phényle éventuellement substitué par des radicaux alkyle en C₁ à C₃ ou des atomes d'halogènes], ou bien, lorsque R¹ = H, un tautomère d'un tel composé, avec une amine de la formule III
HA III,
dans laquelle A possède les significations qui lui ont été attribuées, ou bien
b) on fait réagir une α-halogénocétone de la formule IV 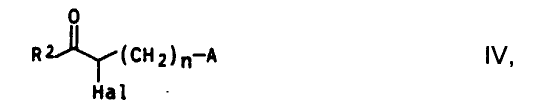 dans laquelle R², R³, A et n possèdent les significations qui leur ont été attribuées dans l'introduction et Hal représente un atome de chlore, de brome ou d'iode, ou un sel d'un acide halogénhydrique de ce composé, avec un thiocarbamate de la formule V 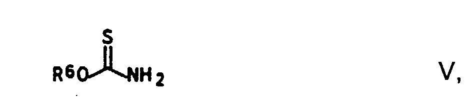 dans laquelle R⁶ représente un radical alkyle en C₁ à C₅, ou avec un tautomère de ce composé, ou bien
c) on alkyle un composé de la formule VI 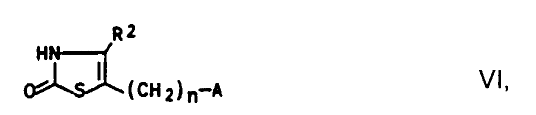 dans laquelle R², A et n possèdent les significations qui leur ont été attribuées dans l'introduction, avec un réactif d'alkylation R¹-X (X = Cl, Br, R⁵SO₂O-) ou R¹₂SO₄ et on convertit les composés ainsi obtenus éventuellement en leurs sels avec des acides physiologiquement compatibles.

3. Thiazoline-2-ones aminoalkylsubstituées à utiliser en vue de lutter contre des maladies.

4. Utilisation d'une thiazoline-2-one aminoalkylsubstituée de la formule I suivant la revendication 1, pour la fabrication d'un médicament contre des maladies du système nerveux central ou des maladies associées à une pression sanguine augmentée.
